# EUROPÄISCHE PATENTSCHRIFT

(11) **EP 2 989 445 B1**
(45) Veröffentlichungstag und Bekanntmachung des Hinweises auf die Patenterteilung: **31.05.2017**
(21) Anmeldenummer: 14711176.9
(22) Anmeldetag: 05.03.2014
(51) Int. Cl.: G01N 21/35, G01N 21/55, A61B 5/145, G01N 21/3563, G01N 21/3577, G01N 21/25, G01N 21/27, A61B 5/00, G01N 21/552

(54) **BILDGEBUNG DURCH ABGESCHWÄCHTE TOTALREFLEXION (ATR)**
IMAGING BY ATTENUATED TOTAL REFLECTANCE (ATR)
IMAGERIE PAR RÉFLEXION TOTALE ATTÉNUÉE (ATR)

(30) Priorität: 21.06.2013 DE 102013211814
(43) Veröffentlichungstag der Anmeldung: 02.03.2016
(73) Patentinhaber: Siemens Aktiengesellschaft, 80333 München (DE)
(72) Erfinder: GEIGER, Florian Benedikt, 81675 München (DE); KÖRDEL, Martin, 80797 München (DE); SCHICK, Anton, 84149 Velden (DE)
(86) Internationale Anmeldenummer: PCT/EP2014/054211
(87) Internationale Veröffentlichungsnummer: WO 2014/202240

(56) Entgegenhaltungen:
- EP-A1- 1 785 089
- EP-A2- 0 516 481
- EP-A2- 0 899 557

## Beschreibung

Die Erfindung betrifft eine Anordnung für die Infrarotspektroskopie in abgeschwächter Totalreflexion (ATR-Infrarotspektroskopie).

In der Infrarotspektroskopie wird unter Verwendung von Licht, dessen Wellenlängenbereich im Infraroten liegt, ein Molekülspektrum einer zu spektroskopierenden Probe gewonnen. In anorganischen und organischen Stoffen wird die Infrarotstrahlung typischerweise durch Anregungen mechanischer Schwingungen eines oder mehrerer Moleküle absorbiert. Auch eine Anregung von Rotationsniveaus bei kleineren Molekülen ist möglich. Da die Schwingungs- bzw. Rotationsenergien als quantenmechanische Eigenzustände charakteristisch für ein Molekül sind, kann aus dem Absorptionsspektrum auf das Molekül und dessen Bindungen geschlossen werden. Die Infrarotspektroskopie ermöglicht daher eine quantitative Strukturaufklärung von Substanzen, deren Identifikation anhand eines Referenzspektrums erfolgt.

Eine besondere Ausführung der Infrarotspektroskopie ist die Infrarotspektroskopie in abgeschwächter Totalreflexion (ATR-Infrarotspektroskopie). Hierbei wird nach dem Stand der Technik die Infrarotstrahlung in einem Reflexionselement durch Totalreflexion geführt. Findet eine solche Totalreflexion an einer Grenzfläche zwischen Probe und Reflexionselement statt, so tritt am Ort der Totalreflexion das infrarote Licht exponentiell in die Probe ein. Diese eintretende evaneszente Welle wechselwirkt mit der Probe, so dass für die Probe charakteristische Frequenzbereiche absorbiert werden. Die absorbierten Frequenzbereiche fehlen daher nun im Spektrum des totalreflektierten Lichtstrahls. Vorteilhafterweise können in der ATR-Infrarotspektroskopie auch Substanzen bzw. Proben spektroskopiert werden, die undurchsichtig für die verwendete Infrarotstrahlung sind und bei denen somit kein Transmissionsspektrum gewonnen werden kann. Zudem eignet sich die ATR-Infrarotspektroskopie für flüssige und/oder pulvrige Proben. Bei vielen Anwendungen in der ATR-Infrarotspektroskopie ist neben der spektralen Information auch eine örtliche Auflösung, d.h. eine Bildgebung der Probe notwendig. Eine solche Bildgebung ist jedoch nach dem Stand der Technik mit starkem Rauschen behaftet. Insbesondere bei Anwendungen der ATR-In-frarotspektroskopie in der Chirurgie ist aber eine hohe Signalstärke und somit eine hohe Trennschärfe von gesundem und krankem Gewebe wünschenswert. Ein weiterer Nachteil bekannter ATR-Infrarotspektroskopien bei der Anwendung in der Chirurgie ist, dass die Proben stets von Flüssigkeiten, insbesondere von Wasser und/oder Blut umgeben sind. Das umgebende Wasser führt aber zu einer starken Absorption im Spektralbereich von etwa 4 µm bis 10 µm, die das eigentliche Messsignal des Gewebes überlagert. EP0899557 A2 offenbart eine ATR- Infrarotspektroskopie. Der vorliegenden Erfindung liegt die Aufgabe zugrunde, eine Anordnung für die ATR-Infrarotspektroskopie anzugeben, die eine signalstarke und ortsaufgelöste Vermessung von wässrigen oder pulvrigen Proben ermöglicht. Weiter ist es Aufgabe der Erfindung, ein entsprechendes Verfahren anzugeben.

Die Aufgabe wird durch eine Anordnung mit den Merkmalen des unabhängigen Anspruchs 1 gelöst. In davon abhängigen Ansprüchen sind vorteilhafte Ausgestaltungen und Weiterbildungen der Erfindung angegeben. Hinsichtlich des Verfahrens wird die Aufgabe durch den Anspruch 10 gelöst. Die erfindungsgemäße Anordnung für die Infrarotspektroskopie in abgeschwächter Totalreflexion umfasst eine Lichtquelle zur Aussendung von Licht, insbesondere infrarotem Licht, Strahlformungskomponenten und einen Detektor zur Detektion eines Abbildes einer zu spektroskopierenden Probe. Weiterhin umfasst die Anordnung eine Reflexionsmatrix, die eine Mehrzahl von Reflexionselementen aufweist, und einen Mehrfachreflexionskristall, der für eine Führung eines Lichtstrahls über innere Totalreflexion ausgebildet ist. Die Reflexionselemente sind in zwei zueinander senkrechten Richtungen einer Fläche regelmäßig angeordnet, so dass diese eine Matrix bilden. Insbesondere kann die Fläche gekrümmt sein. Dadurch sind die Reflexionselemente in einer Richtung senkrecht zu den zwei zueinander senkrechten Richtungen der Fläche gegeneinander versetzt. Weiterhin weisen die einzelnen Reflexionselemente in einem ersten Teilbereich eine konvex geformte Andruckfläche auf, die einer zu spektroskopierenden Probe, insbesondere einer wässerigen oder pulvrigen Probe, zugewandt ist. Die Andruckfläche bildet somit die Grenzfläche zwischen Probe und Reflexionselement aus.

Aus der konvexen Form der Andruckfläche ergeben sich mehrere Vorteile. Zum einen ermöglicht die konvexe Form die Führung des Lichtstrahls über innere Totalreflexion. Zum anderen wird durch die konvexe Form der Andruckfläche, in Kombination mit der regelmäßigen Anordnung als Matrix, eine inkompressible Flüssigkeit, insbesondere Wasser und/oder Blut, beim Andruck auf die Probe in sich durch die konvexe Form ausbildende Kanäle gedrängt. Hierdurch können bei der Infrarotspektroskopie störende Flüssigkeiten, insbesondere Wasser und/oder Blut, durch die sich bildenden Kanäle abfließen. Dadurch wird beispielsweise der störende Einfluss der Wasserabsorption, die im gesamten mittleren infraroten Wellenlängenbereich liegt, insbesondere im Bereich von 4 µm bis 10 µm, signifikant reduziert. Vorteilhafterweise können somit wässrige und/oder pulvrige Proben ohne hohen Präparationsaufwand spektroskopiert werden.

Dies ist besonders bei Anwendungen in der Chirurgie vorteilhaft, wo Gewebeuntersuchungen während einer Operation keine Präparation zulassen und die Gewebe meist von komplexen Flüssigkeiten, insbesondere von Blut, umgeben sind. Zweckmäßig wird das Wasser und/oder Blut auf dem zu spektroskopierenden Gewebe durch das Anpressen der konvexen Andruckflächen verdrängt und durch die sich ausbildenden Kanäle vom Messort entfernt. Vorteilhaft ist zudem die Anordnung der Reflexionselemente an die Oberfläche eines Organes, beispielsweise einer Leber oder Niere, anzupassen. Dadurch gelingt ein bündiger Kontakt zwischen der Reflexionsmatrix und dem zu untersuchenden Organ. Durch die Anpassung sind die Reflexionselemente in einer gekrümmten Fläche, die die Oberfläche eines Organs nachbildet, regelmäßig angeordnet.

Vorteilhafterweise bilden die Reflexionselemente in einer Fläche eine Matrix aus. Durch die Anordnung als Matrix kann jedem Reflexionselement genau ein Bildpunkt bei der ATR-In-frarotspektroskopie zugeordnet werden, so dass zudem eine Ortsauflösung der zu spektroskopierenden Probe ermöglicht wird.

Bei dem erfindungsgemäßen Verfahren zur Infrarotspektroskopie mit der erfindungsgemäßen Anordnung werden die folgenden Schritte durchgeführt:
- Erzeugung eines infraroten Lichtstrahls mit einer Infrarotlichtquelle,
- Eintritt des Lichtstrahls in einen Mehrfachreflexionskristall,
   - Anschließender Eintritt des Lichtstrahls in ein Reflexionselement, wobei der Lichtstrahl mittels des Mehrfachreflexionskristalls über innere Totalreflexion mehrfach zu einem Reflexionselement geführt wird,
   - Detektion des vom Reflexionselement reflektierten Lichtstrahls mit wenigstens einem Infrarotdetektor.

Das Verfahren ermöglicht die Beleuchtung wenigstens eines Reflexionselements und die Detektion des Lichtstrahls, der aus dem Reflexionselement austritt bzw. von diesem reflektiert wird. Dadurch kann ein ATR-Infrarotspektrum einer zu spektroskopierenden Probe gewonnen werden.

In einer vorteilhaften Ausgestaltung können die Reflexionselemente im ersten Teilbereich halbzylinderförmig gestaltet sein. Dadurch bilden sich bei der erfindungsgemäßen Anordnung als Matrix parallele Kanäle aus. Hierbei verlaufen die Kanäle beispielsweise längs einer der zueinander senkrechten Richtungen.

Vorteilhafterweise wird durch die Halbzylinderform eine Totalreflexion des Lichtstrahls ermöglicht.

Besonders vorteilhaft ist, dass inkompressible Flüssigkeiten durch die parallelen Kanäle abfließen können. Insbesondere werden die inkompressiblen Flüssigkeiten von dem Ort der Totalreflexion und somit vom Messort verdrängt.

In einer besonders vorteilhaften Ausgestaltung können die Reflexionselemente im ersten Teilbereich halbkugelförmig gestaltet sein. Dadurch bilden sich bei der erfindungsgemäßen Anordnung als Matrix jeweils parallele Kanäle in den beiden zueinander senkrechten Richtungen der Matrix aus. Hierdurch erhöht sich die Anzahl der Kanäle, so dass sich der Durchsatz an Flüssigkeit erhöht. Besonders vorteilhaft ist, dass durch die halbkugelförmige Ausgestaltung der Lichtstrahl eine Mehrzahl von Totalreflexionen erfahren kann, so dass sich zudem das Signal und somit das Signal- zu Rauschverhältnis erhöht.

In einer besonders vorteilhaften Ausgestaltung können die Reflexionselemente im ersten Teilbereich pyramidenförmig gestaltet sein. Dadurch ergeben sich gleichartige Vorteile wie aus Anspruch 3. Vorteilhafterweise besitzt eine Pyramide eine rechteckige Grundfläche, was den Anschluss an einen zweiten Teilbereich, der quaderförmig ausgebildet sein kann, erleichtert.

Ein zweiter Teilbereich der Reflexionselemente kann quaderförmig ausgebildet sein. Die Quaderform des zweiten Teilbereichs ermöglicht eine einfache und passgenaue bzw. bündige Anordnung der Reflexionselemente als Matrix. Vorteilhaft ist, dass durch den bündigen Abschluss der Reflexionselemente eine inkompressible Flüssigkeit durch die Kanäle entweicht und nicht auf eine der Probe abgewandten Oberfläche der zweiten Teilbereiche geführt wird.

In einer vorteilhaften Ausgestaltung können die Reflexionselemente der Reflexionsmatrix eine spiegelnde Beschichtung, insbesondere zum Spiegeln von Infrarotstrahlung, aufweisen. Dadurch kann die Führung des Lichtstrahls beeinflusst werden, so dass sich eine Mehrzahl von Totalreflexionen ergibt. Hierdurch wird das Signal- zu Rauschverhältnis erhöht. Beispielsweise ist auch eine Kreisführung des Lichtstrahls möglich. Anstatt oder ergänzend zu einer spiegelnden Beschichtung kann auch eine Mehrzahl von Materialien verwendet werden, die unterschiedliche Brechungsindizes besitzen. Durch eine gezielte Abstimmung der Brechungsindizes kann eine gewünschte Führung des Lichtstrahls, die insbesondere eine Mehrzahl von Totalreflexionen des Lichtstrahls aufweist, erreicht werden.

Die Reflexionselemente können wenigstens eines der Materialien ZnSe, Ge, Si oder Diamant umfassen. Durch den hohen Brechungsindex der genannten Materialien wird die interne Totalreflexion des Lichtstrahls ermöglicht. Besonders vorteilhaft ist Ge, das einen Brechungsindex von etwa 4 besitzt. Hierdurch kann eine hohe Auflösung im bildgebenden Verfahren erreicht werden.

Der Mehrfachreflexionskristall ermöglicht die mehrfache Führung des selbigen Lichtstrahls zu einem Reflexionselement. Dadurch ergibt sich eine Mehrzahl von wiederholten Totalreflexionen an der Andruckfläche der Reflexionselemente, so dass die Signalstärke und somit das Signal- zu Rauschverhältnis erhöht wird. In Bezug auf die Probe charakteristische Absorptionsbanden sind dadurch stärker ausgeprägt.

Das Mehrfachreflexionselement kann bezüglich der Reflexionsmatrix verfahrbar angeordnet sein. Dadurch ist es möglich das Mehrfachreflexionselement über ein beliebiges Reflexionselement der Reflexionsmatrix zu platzieren. Die Platzierung kann nacheinander für jedes Reflexionselement durch die Verfahrbarkeit erfolgen, so dass sich vorteilhafterweise eine Ortsauflösung mit hohem Signal- zu Rauschverhältnis ergibt.

In einer vorteilhaften Weiterbildung besitzen das Mehrfachreflexionselement und der zweite Teilbereich der Reflexionselemente im Wesentlichen den gleichen Brechungsindex. Dadurch kann eine sich auf den Strahlengang negativ auswirkende Brechung des Lichtstrahls an der Grenzfläche Mehrfachreflexionselement/Reflexionselement vermieden werden.

Gemäß einer vorteilhaften Ausgestaltung kann der Lichtstrahl entlang der Reflexionsmatrix verfahren werden, so dass jedes einzelne Reflexionselement wenigstens einmal beleuchtet wird. Durch die Verfahrung des Lichtstrahls entlang der Reflexionsmatrix wird ein scannendes Verfahren realisiert. Jedes Reflexionselement der Reflexionsmatrix wird wenigstens einmal beleuchtet, so dass das Verfahren eine Bildgebung der Probe ermöglicht. Vorteilhafterweise korrespondiert ein Bildpunkt jeweils zu einem Messsignal eines Reflexionselements. Es wird somit für jeden Bildpunkt ein ATR-Infrarotspektrum aufgenommen. Besonders vorteilhaft ist, dass durch das Verfahren in Kombination mit der erfindungsgemäßen Reflexionsmatrix eine Bildgebung der Probe mit hoher Signalstärke ermöglicht wird. Insbesondere bei Anwendungen in der Chirurgie ermöglicht das Verfahren eine hinreichende Unterscheidung von Tumorgewebe und gesundem bzw. normalen Gewebe.

In einer vorteilhaften Ausgestaltung kann bei dem Verfahren die Beleuchtung der einzelnen Reflexionselemente durch das Licht der Lichtquelle simultan erfolgen. Dadurch wir ein gesamtes Abbild der Probe gewonnen. Die Ortsauflösung wird anschließend im Detektor durch die Verwendung eines Matrix-Detektors (Focal Plane Array) ermöglicht. Hierbei wird somit für jeden Bildpunkt ein ATR-Infrarotspektrum aufgenommen.

Bei dem Verfahren können die Reflexionselemente an die zu spektroskopierende Probe angedrückt werden. Vorteilhafterweise wird dadurch die Signalstärke erhöht. Besonders vorteilhaft ist, dass sich durch den Andruck der erfindungsgemäßen Reflexionsmatrix Kanäle ausbilden, die einen Abfluss von inkompressiblen Flüssigkeiten, insbesondere von Wasser und/oder Blut, ermöglichen. Hierdurch kann der störende Einfluss, insbesondere von Wasser, auf das Messsignal vermindert werden. Zudem ist vorteilhafterweise keine aufwendige Präparation von wässrigen und/oder pulvrigen Proben nötigt. Dies ist besonders bei Anwendungen in der Chirurgie vorteilhaft, wo Gewebeuntersuchungen während einer Operation keine Präparation zulassen und die Gewebe meist von Flüssigkeiten, insbesondere von Blut, umgeben sind. Der Lichtstahl der Infrarotlichtquelle tritt zuerst in ein Mehrfachreflexionselement und anschließend in ein Reflexionselement ein. Dadurch kann vorteilhafterweise der Lichtstrahl mehrfach zum Messort im Reflexionselement geführt werden, so dass sich das Signal- zu Rauschverhältnis erhöht.

Vorteilhaft ist, wenn der Lichtstrahl wenigstens vier Mal vor der Detektion im Infrarotdetektor in ein Reflexionselement eintritt. Besonders vorteilhaft ist eine Eintrittsanzahl von zehn. Dies kann durch das Mehrfachreflexionselement ermöglicht werden. Die Signalstärke ist in etwa proportional zur Anzahl der Totalreflexionen an der Grenzfläche Reflexionselement/Probe(Andruckfläche). Eine vierfache Totalreflexion an der Andruckfläche kann durch bekannte Mehrfachreflexionselemente einfach realisiert werden.

Die Erfindung wird nachfolgend anhand dreier bevorzugter Ausführungsbeispiele unter Bezugnahme auf die angehängte Zeichnung beschrieben, in der
- Figur 1: eine seitliche Ansicht der Anordnung zur ATR Infrarotspektroskopie zeigt,
- Figur 2: eine dreidimensionale Darstellung der Reflexionselemente und deren Anordnung als Reflexionsmatrix verdeutlicht,
- Figur 3: eine dreidimensionale Darstellung der Reflexionsmatrix mit einem Mehrfachreflexionselement zeigt.

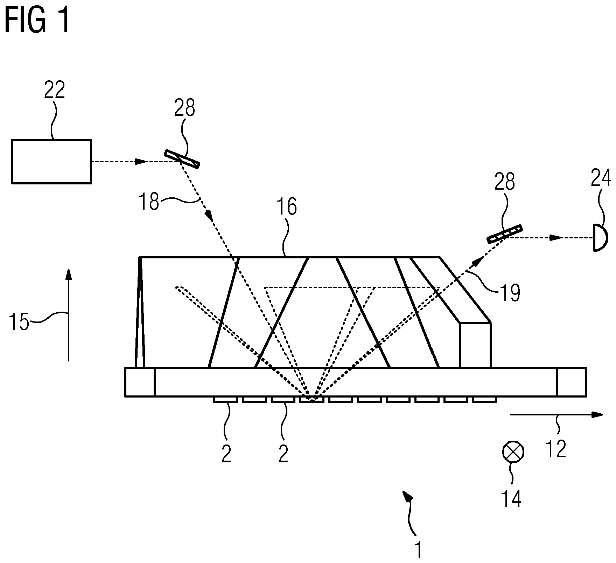

Figur 1 zeigt einen Querschnitt längs einer Richtung 12 der Reflexionsmatrix 1, die einzelne Reflexionselemente 2 aufweist, mit einem Mehrfachreflexionskristall 16. Die in diesem Ausführungsbeispiel gezeigte Anordnung zur ATR-Infrarotspektroskopie umfasst zudem eine Infrarotlichtquelle 22, Spiegel und/oder allgemeine Strahlformungskomponenten 28 und einen Infrarotdetektor 24. Ein infraroter Lichtstrahl 18, ausgehend von der Infrarotlichtquelle 22, trifft zuerst auf den Mehrfachreflexionskristall 16. Der Mehrfachreflexionskristall 16 führt den Lichtstrahl 18 über eine Mehrzahl von inneren Totalreflexionen mehrfach zu einem der Reflexionselemente 2. Anschließend tritt der reflektierte Lichtstrahl 19 aus dem Mehrfachreflexionskristall 16 aus und wird durch Spiegel und/oder allgemeinen Strahlformungskomponenten 28 zum Infrarotdetektor 24 zur Detektion geführt. Der Mehrfachreflexionskristall 16 befindet sich bezüglich einer Richtung 15 oberhalb von der Reflexionsmatrix 1 und ist bezüglich dieser in den zueinander senkrechten Richtungen 12, 14 verfahrbar. Vorteilhafterweise kann dadurch für jedes Reflexionselement 2 der Reflexionsmatrix 1 ein ATR-Infrarotspektrum durch den Detektor 24 aufgenommen werden.

Durch die mehrfache Führung des Lichtstrahls 18, durch das Mehrfachreflexionselement 16, wird die Signalstärke in diesem Ausführungsbeispiel vervierfacht. Dadurch wird eine Bildgebung mit hohem Signal- zu Rauschverhältnis ermöglicht.

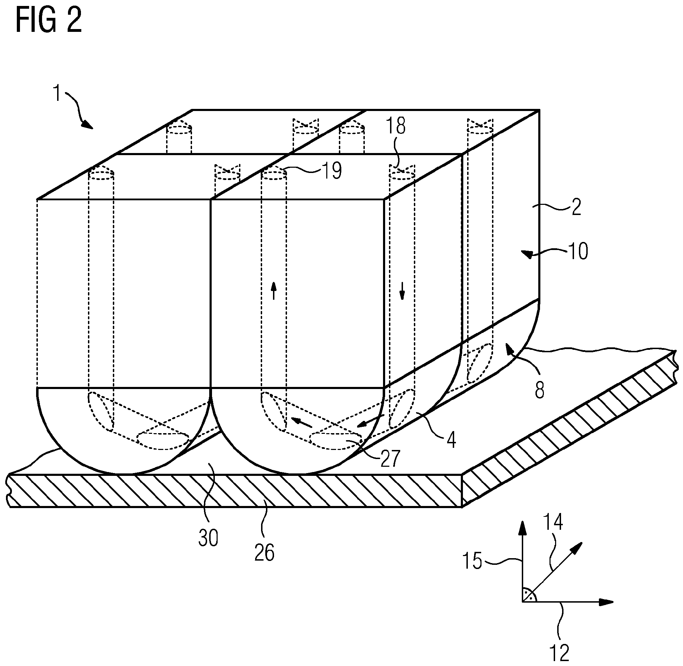

Figur 2 zeigt eine dreidimensionale Anordnung der Reflexionselemente 2. Hierbei sind die Reflexionselemente 2 in den zueinander senkrechten Richtungen 12, 14 regelmäßig angeordnet und bilden die Reflexionsmatrix 1. Typischerweise liegt die Ausdehnung eines Reflexionselements 2 im Bereich von etwa 0,5 mm bis 1,5 mm. Der Lichtstrahl 18, der von der hier nicht gezeigten Infrarotlichtquelle 22 ausgeht, tritt senkrecht bezüglich der zwei Richtungen 12, 14 in einen zweiten Teilbereich 10 der Reflexionselemente 2 ein. Der zweite Teilbereich 10 ist vorteilhafterweise quaderförmig ausgebildet, so dass sich eine bündige Matrixanordnung der Reflexionselemente 2 ergibt. In diesem Ausführungsbeispiel sind die Reflexionselemente 2 in einem ersten Teilbereich 8 halbzylinderförmig ausgebildet. Die Reflexionselemente könnten auch keilförmig ausgebildet sein. Vorteilhafterweise bilden sich durch die konvexe Form einer Andruckfläche 4 Kanäle 30 aus, die den Abfluss von beispielsweise Wasser und/oder Blut ermöglichen. Hierbei wird das Wasser und/oder Blut in der Umgebung der zu spektroskopierenden Probe 26 durch den Andruck der Andruckflächen 4 in die Kanäle 30 gedrängt und von diesen vom Messort 27 abtransportiert. Der aus dem zweiten Teilbereich entlang der Richtung 15 austretende Lichtstrahl 19 kann dann zum hier nicht gezeigten Infrarotdetektor 24 geleitet werden oder durch das nicht gezeigte Mehrfachreflexionselement 16 nochmals als Lichtstrahl 18 zum Reflexionselement 2 zurückgeführt werden.

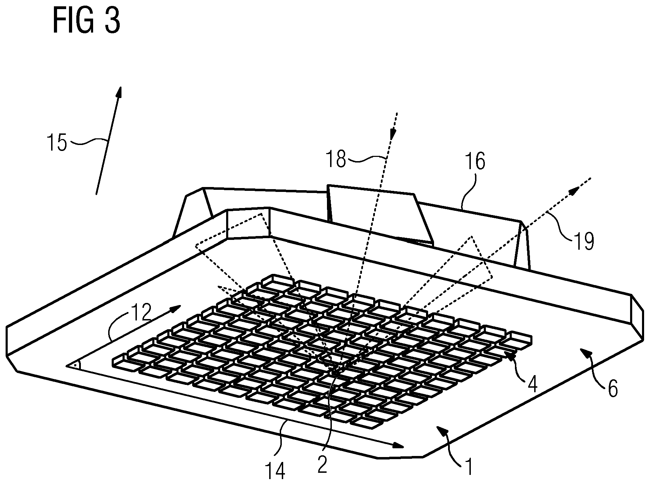

Figur 3 zeigt eine dreidimensionale Darstellung der Reflexionsmatrix 1. Die gezeigte Anordnung umfasst zudem einen Mehrfachreflexionskristall 16. Die einzelnen Reflexionselemente 2 sind in zwei zueinander senkrechten Richtungen 12, 14 in einer planen Ebene 6 regelmäßig angeordnet. Sie bilden somit die Reflexionsmatrix 1. Hierbei befinden sich die Andruckflächen 4 der Reflexionselemente 2 bezüglich der zu den Richtungen 12, 14 senkrechten Richtung 15 vor der Ebene 6, so dass die Andruckflächen 4 auf eine in diesem Ausführungsbeispiel nicht gezeigten Probe 26 angedrückt werden können. Wie bereits in Figur 1 ausgeführt tritt der Lichtstrahl 18 zuerst in das Mehrfachreflexionselement 16 ein, wird mehrfach zu den Reflexionselementen 2 geführt und tritt anschließend als Lichtstrahl 19 wieder aus.

## Patentansprüche

1. Anordnung für die Infrarotspektroskopie in abgeschwächter Totalreflexion, die eine Lichtquelle (22), einen Detektor (24), Strahlformungskomponenten (28), einen Mehrfachreflexionskristall (16) und eine Reflexionsmatrix (1) mit einer Mehrzahl von Reflexionselementen (2) umfasst, wobei die Reflexionselemente (2) zur Führung eines Lichtstrahls (18) über innere Totalreflexion ausgebildet sind,
wobei
eine Andruckfläche (4) in einem ersten Teilbereich (8) der Reflexionselemente (2) konvex geformt ist und die Andruckfläche (4) einer zu spektroskopierenden Probe (26) zugewandt ist, wobei die Reflexionselemente (2) in zwei zueinander senkrechten Richtungen (12, 14) einer Fläche (6) regelmäßig angeordnet sind, **dadurch gekennzeichnet, dass** der Mehrfachreflexionskristall (16) dazu ausgebildet ist, den Lichtstrahl (18) über innere Totalreflexion mehrfach zu einem der Reflexionselemente (2) zu führen.

2. Anordnung nach Anspruch 1, **dadurch gekennzeichnet, dass** die Reflexionselemente (2) im ersten Teilbereich (8) im Wesentlichen halbzylinderförmig ausgebildet sind.

3. Anordnung nach Anspruch 1, **dadurch gekennzeichnet, dass** die Reflexionselemente (2) im ersten Teilbereich (8) im Wesentlichen halbkugelförmig ausgebildet sind.

4. Anordnung nach Anspruch 1, **dadurch gekennzeichnet, dass** die Reflexionselemente (2) im ersten Teilbereich (8) im Wesentlichen pyramidenförmig ausgebildet sind.

5. Anordnung nach einem der vorhergehenden Ansprüche, **dadurch gekennzeichnet, dass** die Reflexionselemente (2) in einem zweiten Teilbereich (10) im Wesentlichen quaderförmig ausgebildet sind.

6. Anordnung nach einem der vorhergehenden Ansprüche, **dadurch gekennzeichnet, dass** die Reflexionselemente (2) eine spiegelnde Beschichtung aufweisen.

7. Anordnung nach einem der vorhergehenden Ansprüche **dadurch gekennzeichnet, dass** die Reflexionselemente (2) eines der Materialien ZnSe, Ge, Si oder Diamant umfassen.

8. Anordnung nach einem der vorhergehenden Ansprüche, **dadurch gekennzeichnet, dass** der Mehrfachreflexionskristall (16) bezüglich der Reflexionsmatrix (1) verfahrbar angeordnet ist.

9. Anordnung nach Anspruch nach einem der vorhergehenden Ansprüche, **dadurch gekennzeichnet, dass** der Mehrfachreflexionskristall (16) und der zweite Teilbereich (10) der Reflexionselemente (2) einen im Wesentlichen gleichen Brechungsindex besitzen.

10. Verfahren zur Infrarotspektroskopie mit einer Reflexionsmatrix gemäß einem der vorhergehenden Ansprüche, das wenigstens folgende Schritte aufweist:
- Erzeugung eines infraroten Lichtstrahls (18) mit einer Infrarotlichtquelle (22),
- Eintritt des Lichtstrahls (18) in einen Mehrfachreflexionskristall (16),
- Anschließender Eintritt des Lichtstrahls (18) in ein Reflexionselement (2), wobei der Lichtstrahl (18) mittels des Mehrfachreflexionskristalls (16) über innere Totalreflexion mehrfach zum Reflexionselement (2) geführt wird,
- Detektion des vom Reflexionselement (2) reflektierten Lichtstrahls (19) mit wenigstens einem Infrarotdetektor (24).

11. Verfahren nach Anspruch 10, bei dem der Lichtstrahl (18) entlang der Reflexionsmatrix (1) verfahren wird, so dass jedes einzelne Reflexionselement (2) wenigstens einmal beleuchtet wird.

12. Verfahren nach Anspruch 10, bei dem eine simultane Beleuchtung von einer Mehrzahl von Reflexionselementen (2) durch den Lichtstrahl (18) erfolgt.

13. Verfahren nach einem der Ansprüche 10-12, bei dem die Andruckflächen (4) der Reflexionselemente (2) an eine zu spektroskopierende Probe (26) angedrückt werden.

14. Verfahren nach einem der Ansprüche 10-13, bei dem der Lichtstrahl (18) wenigstens vier Mal vor der Detektion im Infrarotdetektor (24) in ein Reflexionselement (2) eintritt.

## Claims

1. Arrangement for infrared spectroscopy in attenuated total reflection, which comprises a light source (22), a detector (24), beam shaping components (28), a multiple reflection crystal (16) and a reflection matrix (1) having a multiplicity of reflection elements (2), the reflection elements (2) being configured in order to guide a light beam (18) by means of total internal reflection,
wherein
an application surface (4) in a first subregion (8) of the reflection elements (2) is convexly shaped and the application surface (4) faces toward a sample (26) to be spectroscopically analyzed, the reflection elements (2) being arranged regularly in two mutually perpendicular directions (12, 14) of a surface (6), **characterized in that** the multiple reflection crystal (16) is configured to guide the light beam (18) by means of total internal reflection multiply to one of the reflection elements (2).

2. Arrangement according to Claim 1, **characterized in that** the reflection elements (2) are configured essentially in the shape of a semicylinder in the first subregion (8).

3. Arrangement according to Claim 1, **characterized in that** the reflection elements (2) are configured essentially in the shape of a hemisphere in the first subregion (8).

4. Arrangement according to Claim 1, **characterized in that** the reflection elements (2) are configured essentially in the shape of a pyramid in the first subregion (8).

5. Arrangement according to one of the preceding claims, **characterized in that** the reflection elements (2) are configured essentially in the shape of a cuboid in a second subregion (10).

6. Arrangement according to one of the preceding claims, **characterized in that** the reflection elements (2) have a reflective coating.

7. Arrangement according to one of the preceding claims, **characterized in that** the reflection elements (2) comprise one of the materials ZnSe, Ge, Si or diamond.

8. Arrangement according to one of the preceding claims, **characterized in that** the multiple reflection crystal (16) is arranged displaceably relative to the reflection matrix (1).

9. Arrangement according to one of the preceding claims, **characterized in that** the multiple reflection crystal (16) and the second subregion (10) of the reflection elements (2) have an essentially equal refractive index.

10. Method for infrared spectroscopy with a reflection matrix according to one of the preceding claims, which comprises at least the following steps:
- generation of an infrared light beam (18) with an infrared light source (22),
- entry of the light beam (18) into a multiple reflection crystal (16),
- subsequent entry of the light beam (18) into a reflection element (2), wherein the light beam (18) is guided by means of the multiple reflection crystal (16) by means of total internal reflection multiply to the reflection element (2),
- detection of the light beam (19) reflected by the reflection element (2) with at least one infrared detector (24).

11. Method according to Claim 10, wherein the light beam (18) is displaced along the reflection matrix (1) so that each individual reflection element (2) is illuminated at least once.

12. Method according to Claim 10, wherein simultaneous illumination of a multiplicity of reflection elements (2) by the light beam (18) takes place.

13. Method according to one of Claims 10-12, wherein the application surfaces (4) of the reflection elements (2) are pressed onto a sample (26) to be spectroscopically analyzed.

14. Method according to one of Claims 10-13, wherein the light beam (18) enters a reflection element (2) at least four times before the detection in the infrared detector (24).

## Revendications

1. Agencement de spectroscopie infrarouge en réflexion totale atténuée, qui comprend une source (22) lumineuse, un détecteur (24), un composant (28) de formation d'un faisceau, un cristal (16) à réflexions multiples et une matrice (1) de réflexion ayant une pluralité d'éléments (2) de réflexion, les éléments (2) de réflexion étant constitués pour guider un faisceau (18) lumineux par une réflexion intérieure totale,
dans lequel
une surface (4) d'application d'une pression est formée de manière convexe dans une première sous-partie (8) des éléments (2) de réflexion et la surface (4) d'application d'une pression est tournée vers un échantillon (26) à spectroscopier, les éléments (2) de réflexion étant disposés régulièrement dans deux directions (12, 14) perpendiculaires entre elles d'une surface (6), **caractérisé en ce que** le cristal (16) à réflexions multiples est constitué pour guider le faisceau (18) lumineux par réflexion intérieure totale, plusieurs fois vers l'un des éléments (2) de réflexion.

2. Agencement suivant la revendication 1, **caractérisé en ce que** les éléments (2) de réflexion sont constitués dans la première sous-partie (8), sensiblement sous une forme hémicylindrique.

3. Agencement suivant la revendication 1, **caractérisé en ce que** les éléments (2) de réflexion sont constitués dans la première sous-partie (8), sensiblement sous forme hémisphérique.

4. Agencement suivant la revendication 1, **caractérisé en ce que** les éléments de réflexion sont constitués dans la première sous-partie (8), sensiblement sous forme de pyramide.

5. Agencement suivant l'une des revendications précédentes, **caractérisé en ce que** les éléments (2) de réflexion sont constitués dans une deuxième sous-partie (10) sensiblement sous forme de parallélépipède.

6. Agencement suivant l'une des revendications précédentes, **caractérisé en ce que** les éléments (2) de réflexion ont un revêtement réfléchissant.

7. Agencement suivant l'une des revendications précédentes, **caractérisé en ce que** les éléments (2) de réflexion comprennent l'une des matières ZnSe, Ge, Si ou diamant.

8. Agencement suivant l'une des revendications précédentes, **caractérisé en ce que** le cristal (16) à réflexions multiples est monté de manière à pouvoir être déplacé par rapport à la matrice (1) de réflexion.

9. Agencement suivant l'une des revendications précédentes, **caractérisé en ce que** le cristal (16) à réflexions multiples et la deuxième sous-partie (10) des éléments (2) de réflexion ont sensiblement le même indice de réfraction.

10. Procédé de spectroscopie infrarouge, par une matrice de réflexion suivant l'une des revendications précédentes, qui a au moins les stades suivants :
- production d'un faisceau (18) lumineux infrarouge par une source (22) de lumière infrarouge,
- entrée du faisceau (18) lumineux dans un cristal (16) à réflexions multiples,
- entrée ensuite du faisceau (18) lumineux dans un élément (2) de réflexion, le faisceau (18) lumineux étant guidé au moyen du cristal (16) à réflexions multiples, par réflexion intérieure totale, plusieurs fois vers l'élément (2) de réflexion,
- détection par au moins un détecteur (24) d'infrarouge du faisceau (19) lumineux réfléchi par l'élément (2) de réflexion.

11. Procédé suivant la revendication 10, dans lequel on déplace le faisceau (18) lumineux le long de la matrice (1) de réflexion, de manière à éclairer, au moins une fois, chaque élément (2) de réflexion individuel.

12. Procédé suivant la revendication 10, dans lequel on effectue un éclairage simultané d'une multiplicité d'éléments (2) de réflexion par le faisceau (18) lumineux.

13. Procédé suivant l'une des revendications 10 à 12, dans lequel on applique les surfaces (4) d'application d'une pression des éléments (2) de réflexion sur un échantillon (26) à spectroscopier.

14. Procédé suivant l'une des revendications 10 à 13, dans lequel le faisceau (18) lumineux entre, dans un élément (2) de réflexion, au moins quatre fois avant la détection dans le détecteur (24) d'infrarouge.
